# EUROPÄISCHE PATENTSCHRIFT

(11) **EP 3 202 455 B1**
(45) Veröffentlichungstag und Bekanntmachung des Hinweises auf die Patenterteilung: **26.04.2023**
(21) Anmeldenummer: 17151781.6
(22) Anmeldetag: 17.01.2017
(51) Int. Cl.: A61M 39/10, F16L 19/02, F16L 27/02, F16L 37/084

(54) **MEDIZINISCHE KUPPLUNG UND MEDIZINISCHES SYSTEM MIT EINER MEDIZINISCHEN KUPPLUNG**
MEDICAL COUPLING AND MEDICAL SYSTEM WITH A MEDICAL COUPLING
RACCORD MÉDICAL ET SYSTÈME MÉDICAL COMPRENANT UN RACCORD MÉDICAL

(30) Priorität: 03.02.2016 DE 102016101911
(43) Veröffentlichungstag der Anmeldung: 09.08.2017
(73) Patentinhaber: B. Braun Avitum AG, 34212 Melsungen (DE)
(72) Erfinder: STENZEL, Bruno, 34346 Hann. Münden (DE); BRÖGGER, Sebastian, 34593 Knüllwald (DE); LUDWIG, Uta, 37287 Wehretal (DE)
(74) Vertreter: Winter, Brandl - Partnerschaft mbB

(56) Entgegenhaltungen:
- EP-A1- 1 731 128
- EP-A1- 2 138 202
- EP-A1- 3 202 455
- WO-A2-2006/039501
- GB-A- 2 091 364
- US-A1- 2005 101 939
- US-A1- 2009 188 575
- US-A1- 2010 036 329

## Beschreibung

Die vorliegende Erfindung betrifft eine medizinische Kupplung zum fluiddichten Verbinden zweier fluidführender Abschnitte bei medizinischen Anwendungen, welche vorzugsweise den Anschluss eines extrakorporalen Blutschlauchsystems an einen Dialysator sowie an eine Maschine zur extrakorporalen Blutbehandlung ermöglichen, sowie ein medizinisches System mit einer medizinischen Kupplung.

Bei der extrakorporalen Blutbehandlung oder auch Nierenersatztherapie wird das dem Patienten entnommene Blut außerhalb des Körpers in einem Kreislauf kontinuierlich durch einen Dialysator geleitet und dem Patienten wieder zugeführt. Dafür muss das extrakorporale Blutschlauchsystem, welches das Blut des Patienten führt, mit dem Dialysator verbunden werden. Diese Verbindung wird häufig über einen Luer-Lock-Anschluss realisiert.

Beispielsweise ist aus der DE 60 2004 003 477 T2 ein medizinischer Konnektor zum Verbinden eines Schlauches mit einer medizinischen Vorrichtung bekannt. Dieser Konnektor weist an seinem einen Ende einen Luer-Lock-Anschluss auf, über welchen er mit der medizinischen Vorrichtung verbunden werden kann. An seinem anderen Ende ist der Konnektor fest mit dem Schlauch verbunden bzw. ist der Schlauch in dem Konnektor eingeklebt.

US 2010/0036329 A1 zeigt ein Hämostaseventil zum flüssigkeitsdichten Verbinden eines männlichen Abschnitts und eines weiblichen Abschnitts mittels eines verrastbaren Schnapprings.

GB 2 091 364 A zeigt ein fluiddichtes Verbindungssystem mit einem weiblichen Kupplungsabschnitt und einem männlichen Kupplungsabschnitt, die verjüngt ausgebildet sind und über eine umlaufende radiale Dichtung, die in einer umlaufenden Nut am weiblichen Kupplungsabschnitt positioniert ist, zwischen den verjüngten Oberflächen in Kontakt gebracht und mit einer schraubbaren Mutter gekuppelt werden.

EP 2 138 202 A1 zeigt einen Luer-Lock-Anschluss mit einem männlichen Kopplungsabschnitt und einen weiblichen Kopplungsabschnitt, die durch eine umlaufende radiale Dichtung fluiddicht gekoppelt werden können.

US 2009/0188575 A1 offenbart eine lösbare Schnellkupplung bei der zwei Vorsprünge am männlichen Kupplungsabschnitt mit jeweils einer Rückhalteöffnung in der Hülse axial verrasten.

EP 1 731 128 A1 zeigt eine Schnellkupplung für Spritzen, die einen Fluidkanal der Kupplung je nach Kupplungsposition verschließt oder freigibt. Dazu sind am männlichen Kupplungsabschnitt zwei Einrastpositionen in Form von Vertiefungen vorgesehen, in die eine Rastvorrichtung der Hülse greift und den männlichen Kupplungsabschnitt in axialer Richtung festhält.

WO 2006/039501 A2 zeigt einen als Schnellanschluss ausgeführten Hydrocephalus-Shunt, bei dem eine Hülse mit Rasthaken ausgebildete Arme aufweist, die hinter einen Vorsprung am männlichen Kupplungsabschnitt greifen können.

US 2005/0101939 A1 zeigt eine Schnellkupplung mit einem lösbaren Verriegelungsmechanismus in Form eines verschiebbaren Blechs, der durch Anordnung zweier Federelemente in Kombination mit einem Keil die Kupplung von Hülse und männlichem Kupplungsabschnitt axial sichert bzw. freigibt.

Bei Systemen, bei denen Blutschläuche über eine Luer-Lock-Verbindung an eine medizinische Vorrichtung wie etwa einem Dialysator angeschlossen sind, kann es zu folgenden Problemen kommen. So kommt es häufig zu einem Verdrehen des Blutschlauches und oft folgt eine nicht erkannte Selbstlösung der Schraubverbindung. Dieser Nachteil ist, durch die nicht feste Verbindung des Luer-Lock-Anschlusses des Dialysators mit dem Schlauch, technisch bedingt. Es kann durch die Wärmeausdehnung der Luer-Lock-Anschlüsse zu einem Nachlassen der Reibung bzw. Selbsthemmung des Gewindes kommen. Durch die feste Verbindung des Schlauches am Luer-Lock-Anschluss kann der Schlauch ein permanentes Losdrehmoment auf das Gewinde ausüben. Des Weiteren ist die Güte der Schraubverbindung abhängig vom Bediener. Im Laufe der Therapie muss daher, durch das Bedienpersonal, die Verbindung auf korrekten Sitz geprüft und ggf. korrigiert werden.

Es ist daher die Aufgabe der vorliegenden Erfindung eine Kupplung bereitzustellen, bei der sichergestellt wird, dass sich Schläuche bzw. Blutschläuche nicht verdrehen und die eine vom Anwender drehmomentunabhängige Verbindung schafft, sowie das zeitaufwendige Prüfen und Korrigieren der Verbindung vermeidet.

Diese Aufgabe wird erfindungsgemäß durch eine medizinische Kupplung mit den Merkmalen des Anspruchs 1 sowie durch ein medizinisches System mit den Merkmalen des Anspruchs 6 gelöst. Vorteilhafte Weiterbildungen der Erfindung sind in den Unteransprüchen angegeben.

Die erfindungsgemäße medizinische (Fluid-) Kupplung zum fluiddichten Verbinden (mindestens) zweier fluidführender Abschnitte bei medizinischen Anwendungen ist als axial verrastbare Schnellkupplung ausgebildet und weist einen männlichen Kupplungsabschnitt mit einem durchgängigen ersten Kanal und einen über den männlichen Kupplungsabschnitt schiebbaren weiblichen Kupplungsabschnitt mit einem durchgängigen zweiten Kanal auf. Zudem ist am Außenumfang des männlichen Kupplungsabschnitts und/oder am Innenumfang des weiblichen Kupplungsabschnitts eine vorzugsweise umlaufende Radialdichtung vorgesehen.

Mit der erfindungsgemäßen medizinischen Kupplung ist es somit beispielsweise möglich, eine Fluidleitung und ein medizinisches Gerät, oder auch zwei Fluidleitungen, als Beispiele fluidführender Abschnitte, einfach miteinander zu verbinden. Die Kupplung wird über das Verbinden eines männlichen Kupplungsabschnitts und eines weiblichen Kupplungsabschnitts erzielt. Dabei ist der männliche Kupplungsabschnitt als eine Art zylindrischer Vorsprung mit einem durchgängigen, innenliegenden Kanal ausgebildet. Der weibliche Kupplungsabschnitt ist als eine Art Hülse ausgebildet, in welcher der männliche Kupplungsabschnitt teilweise oder vollständig aufgenommen werden kann. Zum Verbinden wird der weibliche Kupplungsabschnitt in axialer Richtung der Hülse bzw. des zylindrischen Vorsprungs über den männlichen Kupplungsabschnitt geschoben. Dabei verrasten die beiden Kupplungsabschnitte (unlösbar) miteinander. Bei einem Einsatz in der Nierenersatztherapie werden beispielsweise das extrakorporale Blutschlauchsystem und der Dialysator nach der Therapie nicht mehr getrennt. Eine unlösbare Verbindung ist daher nicht nachteilhaft. Zudem stellt die zwischen dem männlichen und dem weiblichen Kupplungsabschnitt ausgebildete Radialdichtung sicher, dass die beiden Kupplungsabschnitte und somit die beiden fluidführenden Abschnitte dichtend miteinander verbunden sind.

Mit der so ausgeführten Kupplung kann schnell und einfach eine Verbindung zweier fluidführender Abschnitte erzielt werden. Es sind dafür keine zusätzlichen Sicherungselemente notwendig. Außerdem wird auf ein zeitaufwändiges Verschrauben oder Verdrehen der beiden Kupplungsabschnitte zueinander verzichtet. Die Verbindung ist somit auch sicherer, da sich die beiden Kupplungsabschnitte nicht selbständig voneinander lösen oder losdrehen können. Zudem ist aufgrund der Radialdichtung eine relative Drehung um die Längsachse der Kupplung des weiblichen Kupplungsabschnitts bezüglich des männlichen Kupplungsabschnitts möglich.

Gemäß einem Aspekt der Erfindung kann der männliche Kupplungsabschnitt einstückig mit einem medizinischen Gerät oder einer medizinischen Vorrichtung, insbesondere einem Dialysator oder einer Maschine zur extrakorporalen Blutbehandlung, ausgebildet sein.

Dadurch sind keine zusätzlichen Bauteile für den Anschluss an das medizinische Gerät notwendig und es ist somit eine einfache, bauteilarme und kostengünstige Konfiguration möglich.

Gemäß einem Aspekt der Erfindung kann der männliche Kupplungsabschnitt einen ersten Befestigungsabschnitt aufweisen.

Gemäß einem Aspekt der Erfindung kann der männliche Kupplungsabschnitt über den ersten Befestigungsabschnitt an einem medizinischen Gerät befestigbar sein.

Gemäß einem Aspekt der Erfindung kann der erste Befestigungsabschnitt des männlichen Kupplungsabschnitts als ein Luer-Lock-Anschluss ausgebildet sein, welcher mit einem Luer-Lock-Anschluss des medizinischen Geräts verbindbar ist.

Gemäß einem Aspekt der Erfindung kann der erste Befestigungsabschnitt des männlichen Kupplungsabschnitts als ein Luer-Lock-Anschluss mit einem weiblichen Innenkegel ausgebildet sein, welcher mit einem Luer-Lock-Anschluss mit einem männlichen Außenkegel des medizinischen Geräts verbindbar ist.

Dadurch, dass der männliche Kupplungsabschnitt über den ersten Befestigungsabschnitt mit dem medizinischen Gerät verbunden wird, ist es möglich, die erfindungsgemäße Kupplung auch für bereits vorhandene medizinische Geräte zu verwenden. Mit anderen Worten kann der männliche Kupplungsabschnitt als eine Art Adapter auf beispielsweise einen Luer-Lock-Anschluss des medizinischen Gerätes geschraubt werden und der männliche Kupplungsabschnitt verbindet somit das medizinische Gerät mit dem weiblichen Kupplungsabschnitt.

Gemäß einem Aspekt der Erfindung kann der erste Befestigungsabschnitt des männlichen Kupplungsabschnitts als ein Innengewindeabschnitt ausgebildet sein, welcher mit einem Außengewindeabschnitt des medizinischen Geräts verbindbar ist.

Somit kann beispielsweise der männliche Kupplungsabschnitt als ein Teil des Deckels des medizinischen Geräts ausgebildet sein, welcher mittels eines Gewindes auf ein Gehäuse des medizinischen Geräts geschraubt werden kann.

Gemäß einem Aspekt der Erfindung kann der weibliche Kupplungsabschnitt einstückig mit einer Fluidleitung ausgebildet sein.

Dadurch sind keine zusätzlichen Bauteile für den Anschluss an die Fluidleitung notwendig und es ist somit eine einfache, bauteilarme und kostengünstige Konfiguration möglich.

Gemäß einem Aspekt der Erfindung kann der weibliche Kupplungsabschnitt einen zweiten Befestigungsabschnitt aufweisen.

Gemäß einem Aspekt der Erfindung kann der weibliche Kupplungsabschnitt über den zweiten Befestigungsabschnitt an einer Fluidleitung befestigbar sein.

Gemäß einem Aspekt der Erfindung kann der zweite Befestigungsabschnitt des weiblichen Kupplungsabschnitts als ein Luer-Lock-Anschluss ausgebildet sein, welcher mit einem Luer-Lock-Anschluss der Fluidleitung verbindbar ist.

Gemäß einem Aspekt der Erfindung kann der zweite Befestigungsabschnitt des weiblichen Kupplungsabschnitts als ein Luer-Lock-Anschluss mit einem männlichen Außenkegel ausgebildet sein, welcher mit einem Luer-Lock-Anschluss mit einem weiblichen Innenkegel der Fluidleitung verbindbar ist.

Dadurch, dass der weibliche Kupplungsabschnitt über den zweiten Befestigungsabschnitt mit der Fluidleitung verbunden wird, ist es möglich, die erfindungsgemäße Kupplung auch für bereits vorhandene Fluidleitungen zu verwenden. Mit anderen Worten kann der weibliche Kupplungsabschnitt als eine Art Konnektor auf beispielsweise einen Luer-Lock-Anschluss der Fluidleitung geschraubt werden und der weibliche Kupplungsabschnitt verbindet somit die Fluidleitung mit dem männlichen Kupplungsabschnitt.

Gemäß der Erfindung weist der männliche Kupplungsabschnitt eine vorzugsweise umlaufende Nut auf, in die ein Teil des weiblichen Kupplungsabschnitts einrastet.

Gemäß der Erfindung sind der männliche Kupplungsabschnitt und der weibliche Kupplungsabschnitt über eine Verriegelungsvorrichtung miteinander verbindbar.

Gemäß der Erfindung ist die Verriegelungsvorrichtung an dem weiblichen Kupplungsabschnitt vorgesehen.

Die Verriegelungsvorrichtung ist so ausgebildet, dass ein über einen Hebel betätigbarer Stift in eine Nut geführt werden kann. Die Verriegelungsvorrichtung ist somit eine sichere Verbindung ohne beispielsweise das Material des weiblichen Kupplungsabschnitts übermäßig zu beanspruchen oder das Dichtungselement zu beschädigen.

Gemäß der Erfindung weist der männliche Kupplungsabschnitt einen Anschlag bzw. eine Schulter auf, welche die Bewegung des weiblichen Kupplungsabschnittes in axialer Richtung begrenzt.

Gemäß der Erfindung können der männliche Kupplungsabschnitt und der weibliche Kupplungsabschnitt durch eine Entriegelungsvorrichtung voneinander gelöst werden.

Gemäß der Erfindung ist die Entriegelungsvorrichtung an dem weiblichen Kupplungsabschnitt vorgesehen.

Gemäß einem Aspekt der Erfindung sind die Verriegelungsvorrichtung und die Entriegelungsvorrichtung gleichartig ausgebildet.

Es ist somit möglich, durch einen Entriegelungstaster oder -hebel die durch Einrasten miteinander verbundenen männlichen und weiblichen Kupplungsabschnitte wieder zerstörungsfrei voneinander zu lösen. Alternativ kann das Verbinden und Lösen auch über die Ver- bzw. Entriegelungsvorrichtung realisiert werden.

Das erfindungsgemäße medizinische System enthält eine erste Fluidleitung und ein medizinisches Gerät und/oder einer zweiten Fluidleitung. In der ersten Fluidleitung ist ein erster Kanal und in dem medizinischen Gerät und/oder der zweiten Fluidleitung ist ein zweiter Kanal vorgesehen. Die erste Fluidleitung und das medizinische Gerät und/oder die zweite Fluidleitung können über eine erfindungsgemäße medizinische Kupplung miteinander verbunden werden, sodass der erste Kanal und der zweite Kanal dichtend miteinander verbunden sind.

In dem medizinischen System dient die medizinische Kupplung dazu, mindestens zwei fluidführende Abschnitte miteinander zu verbinden bzw. eine Verbindung herzustellen, sodass ein Fluid von einer Leitung in ein medizinisches Gerät oder eine weitere Leitung strömen bzw. fließen kann oder umgekehrt. Die medizinische Kupplung kann dabei einen oder mehrere der vorstehend beschriebenen Aspekte bzw. Vorteile enthalten.

Zusammenfassend kann mit der erfindungsgemäßen medizinischen Kupplung und dem erfindungsgemäßen medizinischen System, besonders beim Einsatz in der Nierenersatztherapie, die Benutzerfreundlichkeit verbessert werden und es können Rüstzeiten verkürzt werden. Zudem wird ein Verdrehen der Blutschläuche verhindert und eine drehmomentunabhängige Verbindung wird realisiert, welche sich während der Therapie nicht selbstständig lösen kann. Dadurch entfällt das zeitaufwendige Prüfen und Korrigieren der Verbindung durch den Bediener, es kommt zu keinem unbemerkten Blutverlust, was zu einem Patientenrisiko führen könnte, und eine Fehlmessung der Drücke während der Therapie kann verhindert werden.

### Kurze Beschreibung der Zeichnungen

Fig. 1 zeigt eine Schnittansicht eines Adapters mit daran gekoppelter Kupplungshülse gemäß einer nicht beanspruchten ersten Ausführungsform.
Fig. 2 zeigt eine Schnittansicht eines medizinischen Geräts mit daran gekoppelter Kupplungshülse gemäß einer nicht beanspruchten zweiten Ausführungsform.
Fig. 3 zeigt eine Schnittansicht eines Adapters mit daran gekoppelter Kupplungshülse, welche eine Entriegelungsvorrichtung enthält, gemäß einer erfindungsgemäßen dritten Ausführungsform.

### Detaillierte Beschreibung bevorzugter Ausführungsformen

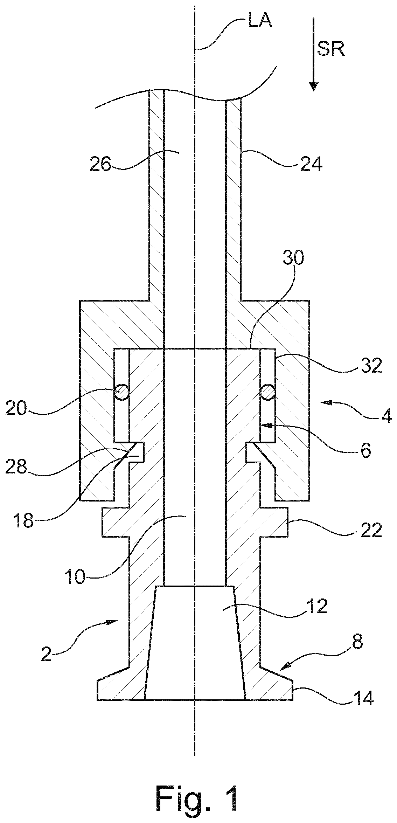

Fig. 1 zeigt eine nicht beanspruchte erste Ausführungsform einer medizinischen Kupplung, bei der ein im Wesentlichen zylindrischer Adapter 2 und eine Kupplungshülse 4 miteinander verbunden sind. Der Adapter 2 hat an seinem der Kupplungshülse 4 zugewandten Ende einen männlichen Kupplungsabschnitt bzw. ersten Kupplungsabschnitt 6 und an dem anderen, der Kupplungshülse 4 abgewandten Ende einen ersten Befestigungsabschnitt 8. Zudem weist der Adapter 2 einen durchgängigen ersten Kanal 10 auf, welcher sich in Richtung weg von der Kupplungshülse 4 kegelförmig aufweitet. Mit anderen Worten wird ein erster weiblicher Luer-Lock-Innenkegel 12 ausgebildet. Weiterhin ist an dem der Kupplungshülse 4 abgewandten Ende des ersten Befestigungsabschnitts 8 ein erster Außengewindeabschnitt bzw. erster Gewindeabschnitt 14 ausgebildet. Über den ersten weiblichen Luer-Lock-Innenkegel 12 und den ersten Gewindeabschnitt 14 ist der Adapter 2 an einem (in Fig. 1 nicht dargestellten) medizinischen Gerät bzw. Vorrichtung 16, wie etwa einem Dialysator oder einer Maschine zur extrakorporalen Blutbehandlung, befestigbar. Im Falle eines Dialysators werden die blutführenden Leitungen angeschlossen und im Falle einer Maschine zur extrakorporalen Blutbehandlung werden die von den blutführenden Leitungen abgehenden Druckleitungen angeschlossen, die eine Luftsäule an die Druckaufnehmer leiten. Mit anderen Worten ist der Adapter 2 an jedem komplementär ausgebildeten Luer-Lock-Anschluss mit männlichem Außenkegel befestigbar.

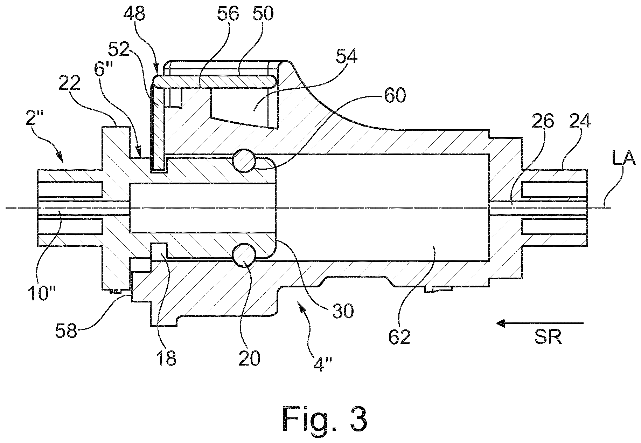

Der erste Kupplungsabschnitt 6 weist ferner eine umlaufende Nut 18 auf. Zudem ist zwischen der Nut 18 und dem der Kupplungshülse 4 zugewandten Ende des ersten Kupplungsabschnitts 6 ein O-Ring 20 als Dichtungselement angeordnet. Der O-Ring 20 kann dabei am Außenumfang des ersten Kupplungsabschnitts 6 anliegen, in einer zusätzlichen, in Fig. 3 dargestellten Dichtungsnut aufgenommen sein oder als Dichtungselement einstückig, z.B. durch einen 2K-Spritzgussprozess, mit dem ersten Kupplungsabschnitt 6 ausgebildet sein. Zusätzlich ist an dem der Kupplungshülse 4 abgewandten Ende des ersten Kupplungsabschnitts 6 bzw. zwischen dem ersten Kupplungsabschnitt 6 und dem ersten Befestigungsabschnitt 8 eine Schulter 22 ausgebildet, welche sich in radialer Richtung nach Außen erstreckt.

Die Kupplungshülse 4 fungiert als weiblicher Kupplungsabschnitt und ist einstückig mit einer Fluidleitung bzw. einem Schlauch 24 ausgebildet, welcher sich an dem von dem Adapter 2 entfernten Ende der Kupplungshülse 4 anschließt. Im Inneren des Schlauches 24 und der Kupplungshülse 4 ist ein zweiter Kanal 26 ausgebildet. Weiterhin sind auf der Innenfläche der Kupplungshülse 4 mehrere, gleichmäßig in der Umfangsrichtung verteilte Schnapphaken 28 ausgebildet. Es ist jedoch auch möglich, dass nur ein einziger umlaufender Schnapphaken ausgebildet ist.

Wird nun die Kupplungshülse 4 in Richtung einer Schieberichtung SR, d.h. entlang einer Längsachse LA des Adapters 2 und der Kupplungshülse 4 in Richtung auf den Adapter 2 zu, auf den Adapter 2 geschoben, dann stoßen die Schnapphaken 28 auf eine der Kupplungshülse 4 zugewandte Endfläche 30 des Adapters 2. Dies führt dazu, dass sich die aus Kunststoff ausgebildete Kupplungshülse 4 in radialer Richtung aufweitet und die Kupplungshülse 4 weiter in der Schieberichtung SR auf den Adapter 2 bzw. den ersten Kupplungsabschnitt 6 geschoben werden kann. Sobald die Schnapphaken 28 in axialer Richtung, d.h. entlang der Längsachse LA, auf Höhe der Nut 18 sind, rasten diese in die Nut 18 ein und die Kupplungshülse 4 kehrt in ihre ursprüngliche, nicht in radialer Richtung aufgeweitete Position zurück. Dabei kommt der O-Ring 20 des Adapters 2 auf einer Dichtungsfläche 32 auf der Innenseite der Kupplungshülse 4 zum Anliegen. Dadurch wird ein dichter Strömungspfad zwischen dem ersten Kanal 10 des Adapters 2 und dem zweiten Kanal 26 der Kupplungshülse 4 bzw. des Schlauchs 24 ausgebildet. Weiterhin begrenzt die Schulter 22 die Bewegung der Kupplungshülse 4 in der Schieberichtung SR, sodass die Schnapphaken 28 in der Nut 18 verbleiben und somit die Kupplungshülse 4 sicher mit dem Adapter 2 verbunden ist. Jedoch ist ein gewisses axiales Spiel zwischen dem Adapter 2 und der Kupplungshülse 4 erlaubt, solange die Schnapphaken 28 nicht aus der Nut 18 herausbewegt werden und der O-Ring 20 auf der Dichtungsfläche 32 verbleibt.

Mit der Kupplung bzw. Schnellkupplung kann nicht nur schnell eine dichte und kraftunabhängige Verbindung zweier fluidführender Abschnitte wie der Kupplungshülse 4 mit Schlauch 24 und dem Adapter 2 hergestellt werden, sondern es ist aufgrund der Radialdichtung mittels O-Ring 20 auch möglich, dass sich die Kupplungshülse 4 und der Adapter 2 relativ zueinander um die Längsachse LA drehen können. Dadurch wird ein Verdrehen des Schlauchs 24 oder auch ein Lösen der Luer-Lock-Verbindung zwischen dem Adapter 2 und dem medizinischen Gerät 16 verhindert. Dabei ist es auch möglich, dass das Dichtungselement in der Kupplungshülse 4 vorgesehen ist und nach dem Verbinden mit einer Dichtungsfläche des Adapters 2 in Kontakt kommt.

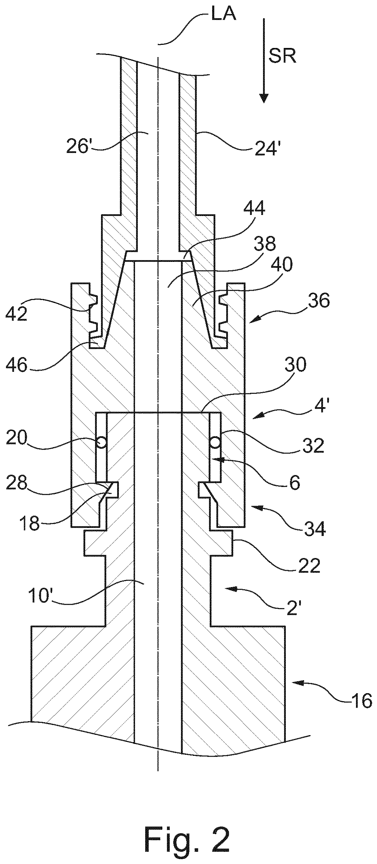

Fig. 2 zeigt eine nicht beanspruchte zweite Ausführungsform einer medizinischen Kupplung. Gleiche Bauteile wie die der ersten Ausführungsform sind dabei mit denselben Bezugszeichen versehen.

In der zweiten Ausführungsform ist der Adapter 2' einstückig mit dem medizinischen Gerät 16 ausgebildet. Mit anderen Worten ist der Adapter 2' vielmehr ein Fortsatz des medizinischen Geräts 16, an dessen der Kupplungshülse 4' zugewandten Ende der erste Kupplungsabschnitt 6 als männlicher Kupplungsabschnitt ausgebildet ist.

In der zweiten Ausführungsform fungiert die Kupplungshülse 4' als Konnektor und verbindet so den Schlauch 24' mit dem medizinischen Gerät 16. Die Kupplungshülse 4' enthält an ihrem, dem medizinischen Gerät 16 zugewandten Ende einen weiblichen Kupplungsabschnitt bzw. zweiten Kupplungsabschnitt 34 und an ihrem, dem Schlauch 24' zugewandten Ende einen zweiten Befestigungsabschnitt 36. Die Verbindung von Adapter 2' und Kupplungshülse 4' bzw. erstem Kupplungsabschnitt 6 und zweiten Kupplungsabschnitt 34 erfolgt analog der ersten Ausführungsform.

In der Kupplungshülse 4' ist außerdem entlang der Längsachse LA ein durchgängiger dritter Kanal 38 ausgebildet. Der zweite Befestigungsabschnitt 36 ist als Luer-Lock-Anschluss ausgebildet. D.h., der dritte Kanal 38 wird von einem sich in Richtung des Schlauchs 24' erstreckenden männlichen Luer-Lock-Außenkegel 40 umschlossen. Zudem ist am radial äußeren Ende des zweiten Befestigungsabschnitts 36 ein Innengewindeabschnitt bzw. zweiter Gewindeabschnitt 42 vorgesehen.

Das der Kupplungshülse 4' zugewandte Ende des Schlauchs 24' ist als ein komplementär zum Luer-Lock-Anschluss der Kupplungshülse 4' ausgebildeter Luer-Lock-Anschluss ausgebildet. So weitet sich der zweite Kanal 26' in Richtung zu der Kupplungshülse 4' hin kegelförmig auf. Mit anderen Worten wird ein zweiter weiblicher Luer-Lock-Innenkegel 44 ausgebildet. Weiterhin ist an dem der Kupplungshülse 4' abgewandtem Ende des Schlauchs 24' ein zweiter Außengewindeabschnitt bzw. dritter Gewindeabschnitt 46 ausgebildet.

Werden nun der erste Kupplungsabschnitt 6 und die Kupplungshülse 4' wie vorstehend beschrieben gekoppelt und werden zusätzlich der Schlauch 24' und die Kupplungshülse 4' über die Luer-Lock-Verbindung miteinander verbunden, kann ein dichter Strömungspfad von dem zweiten Kanal 26' im Schlauch 24' über den dritten Kanal 38 in der Kupplungshülse 4' zu dem ersten Kanal 10' im medizinischen Gerät 16 hergestellt werden.

In den vorstehend beschriebenen Ausführungsformen werden verschiedene Möglichkeiten gezeigt, wie der Schlauch 24 und das medizinische Gerät 16 miteinander verbunden werden können. Es sind jedoch auch weitere Kombinationsmöglichkeiten der verschiedenen Verbindungen möglich. So kann beispielsweise der Adapter 2 einerseits über eine Luer-Lock-Verbindung an dem medizinischen Gerät befestigt und andererseits mit der Kupplungshülse 4', welche über eine Luer-Lock-Verbindung an dem Schlauch 24' befestigt ist, verrastbar verbunden sein.

Fig. 3 zeigt eine erfindungsgemäße dritte Ausführungsform einer medizinischen Kupplung. Gleiche Bauteile wie die der ersten und zweiten Ausführungsform sind dabei mit denselben Bezugszeichen versehen.

In der dritten Ausführungsform weist die Kupplungshülse 4" zusätzlich eine Entriegelungsvorrichtung 48 auf, welche aus einem Hebel 50 und einem Stift 52 besteht. In dem in Fig. 3 gezeigten verriegelten Zustand ist der Hebel 50 parallel zu der Längsachse LA und der Stift 52 senkrecht zur Längsachse LA angeordnet. Dabei ist der Hebel 50 seinem dem Adapter 2" zugewandeten Ende mit einem dem Adapter 2" abgewandeten Ende des Stiftes 52 gelenkig verbunden. Ferner ist die Entriegelungsvorrichtung 48 in einer Aussparung 54 der Kupplungshülse 4" vorgesehen und über eine Anlenkstelle 56 gelenkig mit der Kupplungshülse 4" verbunden.

Um die Kupplungshülse 4" mit dem Adapter 2" verbinden zu können, muss der Hebel 50 an seinem dem Adapter 2" abgewanden Ende in radialer Richtung nach Innen bewegt werden. Dadurch wird der Stift 52 in radialer Richtung nach Außen bewegt und die Kupplungshülse 4" kann in der Schieberichtung SR auf den Adapter 2" bzw. den ersten Kupplungsabschnitt 6" geschoben werden. Sobald eine dem Adapter 2" zugewandte vordere Begrenzungsfläche 58 der Kupplungshülse 4" gegen die Schulter 22 stößt, befinden sich die Nut 18 und der Stift 52 in axialer Richtung auf gleicher Höhe. Wenn nun der Hebel 50 losgelassen d.h. nicht mehr betätigt wird, kehrt der Hebel in seine Ausgangsposition parallel zur Längsachse LA zurück und der Stift 52 schiebt sich in die Nut 18, sodass der Adapter 2" und die Kupplungshülse 4" miteinander verbunden sind. Um die Verbindung zu lösen, muss wiederum der Hebel 50 betätigt, d.h. in radialer Richtung nach Innen bewegt werden. Dadurch bewegt sich der Stift 52 aus der Nut 18 heraus und die Kupplungshülse 4" kann entgegen der Schieberichtung SR von dem Adapter 2" abgezogen werden.

Die Kupplung ist somit in axialer Richtung lösbar ausgeführt. Es ist jedoch auch möglich, dies anstelle über einen Entriegelungshebel über einen Entriegelungstaster oder dergleichen zu realisieren. In der vorstehend beschriebenen dritten Ausführungsform ist die Entriegelungsvorrichtung 48 an der Kupplungshülse 4" angeordnet. Es ist jedoch auch möglich, die Entriegelungsvorrichtung 48 an dem Adapter 2"anzuordnen.

Nachfolgend werden einige Unterschiede bzw. vorteilhafte Ausgestaltungen der dritten Ausführungsform gegenüber der ersten und zweiten Ausführungsform beschrieben.

In der dritten Ausführungsform ist der O-Ring 20 in einer Dichtungsnut 60 aufgenommen. Dadurch wird die radiale Ausdehnung des Dichtungselements begrenzt. Diese Konfiguration kann auch in der ersten und zweiten Ausführungsform verwendet werden, da so vorteilhafterweise die Schnapphaken 28 beim Aufschieben der Kupplungshülse besser über den O-Ring 20 gleiten können.

Außerdem liegt in der dritten Ausführungsform die Endfläche 30 des Adapters 2" nicht direkt an der Kupplungshülse 4". Vielmehr schließt sich dem zweiten Kanal 26 in Richtung zu dem Adapter 2" hin eine radial nach Außen aufgeweitete Aufnahmeöffnung 62 an, in welcher der erste Kupplungsabschnitt 6" aufgenommen werden an. Auch der erste Kanal 10" ist in Richtung zu der Kupplungshülse 4" hin in radialer Richtung nach Außen hin aufgeweitet.

Weiterhin muss in der dritten Ausführungsform nicht nur eine Verriegelung- bzw. Entriegelungsvorrichtung 48 vorgesehen sein. Es können auch zwei oder mehrere vorzugsweise gleichmäßig in der Umfangsrichtung verteilt sein. Zudem kann der Adapter 2" der dritten Ausführungsform entweder analog zu dem Adapter 2 der ersten Ausführungsform an dem medizinischen Gerät 16 befestigbar sein oder analog zu dem Adapter 2' der zweiten Ausführungsform einstückig mit dem medizinischen Gerät 16 ausgebildet sein.

## Patentansprüche

1. Medizinische Kupplung zum flüssigkeitsdichten Verbinden zweier fluidführender Abschnitte (24') bei medizinischen Anwendungen, wobei
die Kupplung als axial Schnellkupplung ausgebildet ist und einen männlichen Kupplungsabschnitt (6"), der als zylindrischer Vorsprung mit einem durchgängigen, innenliegenden ersten Kanal (10") ausgebildet ist, und einen über den männlichen Kupplungsabschnitt (6") schiebbaren weiblichen Kupplungsabschnitt (4") mit einem durchgängigen zweiten Kanal (26) aufweist;
der männliche Kupplungsabschnitt (6") und der weibliche Kupplungsabschnitt (4") miteinander verrastbar sind; und
am Außenumfang des männlichen Kupplungsabschnitts (6") und/oder am Innenumfang des weiblichen Kupplungsabschnitts (4") eine Radialdichtung (20) vorgesehen ist,
**dadurch gekennzeichnet, dass**
der männliche Kupplungsabschnitt (6") eine Nut (18) aufweist, und
der männliche Kupplungsabschnitt (6") an einem dem weiblichen Kupplungsabschnitt (4") abgewandten Ende einen Anschlag oder eine Schulter (22) aufweist, die sich in radialer Richtung nach Außen streckt und dazu vorgesehen ist, die Bewegung des weiblichen Kupplungsabschnitts (4") in axialer Richtung zu begrenzen,
wobei der männliche Kupplungsabschnitt (6") und der weibliche Kupplungsabschnitt (4") durch eine Entriegelungsvorrichtung (48) miteinander verbindbar oder voneinander lösbar sind, wobei die Entriegelungsvorrichtung (48) in einem verriegelten Zustand einen Hebel (50) parallel zu einer Längsachse (LA) der medizinischen Kupplung und einen Stift (52) senkrecht zur Längsachse (LA) aufweist,
wobei der Hebel (50) an seinem dem männlichen Kupplungsabschnitt (6") zugewandeten Ende gelenkig mit einem radial äußeren Ende des Stiftes (52) verbunden ist,
wobei der Stift (52) durch eine gelenkige Bewegung des Hebels (50) über eine Anlenkstelle (56) betätigbar ist, um sich in die Nut (18) zu schieben oder sich aus der Nut (18) heraus zu bewegen, so dass der männliche Kupplungsabschnitt (6") und der weibliche Kupplungsabschnitt (4") miteinander verbindbar oder voneinander lösbar sind, und
wobei die Entriegelungsvorrichtung (48) an dem weiblichen Kupplungsabschnitt (4") vorgesehen ist.

2. Medizinische Kupplung gemäß Anspruch 1, wobei
der männliche Kupplungsabschnitt (6") einstückig mit einem medizinischen Gerät (16) ausgebildet ist.

3. Medizinische Kupplung gemäß Anspruch 1, wobei
der männliche Kupplungsabschnitt (6") einen ersten Befestigungsabschnitt (8) aufweist, und wobei
der männliche Kupplungsabschnitt (6") über den ersten Befestigungsabschnitt (8) an einem medizinischen Gerät (16) befestigbar ist.

4. Medizinische Kupplung gemäß Anspruch 3, wobei
der erste Befestigungsabschnitt (8) des männlichen Kupplungsabschnitts (6") als ein Luer-Lock-Anschluss ausgebildet ist, welcher mit einem Luer-Lock-Anschluss des medizinischen Geräts (16) verbindbar ist.

5. Medizinische Kupplung gemäß einem der Ansprüche 1 bis 4, wobei
der weibliche Kupplungsabschnitt (4") einstückig mit einer Fluidleitung (24) ausgebildet ist.

6. Medizinisches System, mit:
einer ersten Fluidleitung (24) und
einem medizinischen Gerät (16) und/oder einer zweiten Fluidleitung, wobei
in der ersten Fluidleitung (24) ein erster Kanal (26) und in dem medizinischen Gerät (16) und/oder der zweiten Fluidleitung ein zweiter Kanal (10") vorgesehen ist, und wobei
die erste Fluidleitung (24) und das medizinische Gerät (16) und/oder die zweite Fluidleitung über eine medizinische Kupplung gemäß einem der Ansprüche 1 bis 5 miteinander verbunden werden können, sodass der erste Kanal (26) und der zweite Kanal (10") dichtend miteinander verbunden sind.

## Claims

1. A medical coupling for fluid-tight connection of two fluid-guiding portions (24') in medical applications, wherein
the coupling is an axially lockable quick-connect coupling and comprises a male coupling portion (6") configured as a cylindrical projection having a continuous, internal first passage (10"), and a female coupling portion (4") adapted to be slipped over the male coupling portion (6") and having a continuous second passage (26), and wherein
the male coupling portion (6") and the female coupling portion (4") are lockable to each other; and
a circumferential radial sealing (20) is provided on the outer periphery of the male coupling portion (6") and/or on the inner periphery of the female coupling portion (4"),
**characterized in that**
the male coupling portion (6") has a groove (18), and
the male coupling portion (6") has, at an end remote from the female coupling portion (4"), a stop or shoulder (22) which extends outwards in the radial direction and is provided to limit the movement of the female coupling portion (4") in the axial direction,
wherein the male coupling portion (6") and the female coupling portion (4") are connectable to or disconnectable from each other by a release device (48), wherein the release device (48) in a locked state comprises a lever (50) parallel to a longitudinal axis (LA) of the medical coupling and a pin (52) perpendicular to the longitudinal axis (LA),
wherein the lever (50) is hingedly connected at its end facing the male coupling portion (6") to a radially outer end of the pin (52),
wherein the pin (52) is actuatable by an articulated movement of the lever (50) via an articulation point (56) in order to push into or move out of the groove (18), so that the male coupling portion (6") and the female coupling portion (4") are connectable to or disconnectable from each other, and
wherein the unlocking device (48) is provided on the female coupling portion (4").

2. The medical coupling according to claim 1, wherein
the male coupling portion (6") is formed integrally with a medical apparatus (16).

3. The medical coupling according to claim 1, wherein
the male coupling portion (6") includes a first fastening portion (8), and wherein
the male coupling portion (6") is adapted to be fastened to a medical apparatus (16) via the first fastening portion (8).

4. The medical coupling according to claim 3, wherein
the first fastening portion (8) of the male coupling portion (6") is a Luer lock, which is connectable to a Luer lock of the medical apparatus (16).

5. The medical coupling according to one of the claims 1 to 4, wherein
the female coupling portion (4") is formed integrally with a fluid line (24).

6. A medical system comprising:
a first fluid line (24), and
a medical apparatus (16) and/or a second fluid line, wherein
in the first fluid line (24), a first passage (26) is provided and in the medical apparatus (16) and/or the second fluid line a second passage (10") is provided, and wherein
the first fluid line (24) and the medical apparatus (16) and/or the second fluid line can be connected to each other via a medical coupling according to one of the claims 1 to 5 so that the first passage (26) and the second passage (10") are sealed to each other.

## Revendications

1. Couplage médical destiné à la liaison étanche aux liquides de deux sections conductrices de fluide (24') dans des applications médicales, dans lequel
le couplage est réalisé en tant que couplage rapide axial et présente une section de couplage mâle (6") qui est réalisée en tant que saillie cylindrique avec un premier canal intérieur traversant (10") et une section de couplage femelle (4") déplaçable via la section de couplage mâle (6") avec un second canal traversant (26) ;
la section de couplage mâle (6") et la section de couplage femelle (4") peuvent être enclenchées l'une avec l'autre ; et
au niveau de la périphérie extérieure de la section de couplage mâle (6") et/ou au niveau de la périphérie intérieure de la section de couplage femelle (4") un joint d'étanchéité radial (20) est prévu,
**caractérisé en ce que**
la section de couplage mâle (6") présente une rainure (18), et
la section de couplage mâle (6") présente au niveau d'une extrémité opposée à la section de couplage femelle (4") une butée ou un épaulement (22) qui s'étend vers l'extérieur dans la direction radiale et est prévu(e) pour limiter le mouvement de la section de couplage femelle (4") dans la direction axiale,
dans lequel la section de couplage mâle (6") et la section de couplage femelle (4") peuvent être reliées l'une à l'autre et séparées l'une de l'autre de manière amovible par le biais d'un dispositif de déverrouillage (48), dans lequel le dispositif de déverrouillage (48) présente dans un état verrouillé un levier (50) parallèle à un axe longitudinal (LA) du couplage médical et une tige (52) perpendiculaire à l'axe longitudinal (LA),
dans lequel le levier (50) est relié au niveau de son extrémité orientée vers la section de couplage mâle (6") de manière articulée avec une extrémité extérieure radiale de la tige (52),
dans lequel la tige (52) peut être actionnée par le biais d'un mouvement articulé du levier (50) via un point d'articulation (56) pour entrer dans la rainure (18) ou pour sortir de la rainure (18), de sorte que la section de couplage mâle (6") et la section de couplage femelle (4") peuvent être reliées l'une à l'autre ou être séparées l'une de l'autre, et
dans lequel le dispositif de déverrouillage (48) est prévu au niveau de la section de couplage femelle (4").

2. Couplage médical selon la revendication 1, dans lequel
la section de couplage mâle (6") est réalisée d'un seul tenant avec l'appareil médical (16).

3. Couplage médical selon la revendication 1, dans lequel
la section de couplage mâle (6") présente une première section de fixation (8), et dans lequel
la section de couplage mâle (6") peut être fixée à un appareil médical (16) via la première section de fixation (8).

4. Couplage médical selon la revendication 3, dans lequel
la première section de fixation (8) de la section de couplage mâle (6") est réalisée en tant que raccord Luer-Lock qui peut être relié à un raccord Luer-Lock de l'appareil médical (16).

5. Couplage médical selon l'une quelconque des revendications 1 à 4, dans lequel
la section de couplage femelle (4") est réalisée d'un seul tenant avec une conduite de fluide (24).

6. Système médical, avec :
une première conduite de fluide (24) et
un appareil médical (16) et/ou une seconde conduite de fluide, dans lequel
dans la première conduite de fluide (24) un premier canal (26) est prévu et dans l'appareil médical (16) et/ou la seconde conduite de fluide un second canal (10") est prévu, et dans lequel
la première conduite de fluide (24) et l'appareil médical (16) et/ou la seconde conduite de fluide peuvent être reliés les uns aux autres via un couplage médical selon l'une quelconque des revendications 1 à 5, de sorte que le premier canal (26) et le second canal (10") sont reliés l'un à l'autre de manière étanche.
